# EUROPEAN PATENT APPLICATION

(11) **EP 4 205 730 A1**
(43) Date of publication of application: **05.07.2023**
(21) Application number: 21862057.3
(22) Date of filing: 25.08.2021
(51) Int. Cl.: A61K 9/20, A61K 31/4164, A61K 31/4422, A61K 31/505, A61K 9/28, A61K 9/16

(54) **PHARMACEUTICAL COMPOSITION OF SINGLE DOSAGE FORM FOR TREATING OR PREVENTING HYPERTENSION AND HYPERLIPIDEMIA**

(30) Priority: 25.08.2020 KR 20200107083
(71) Applicant: Daewoong Pharmaceutical Co., Ltd., Hwaseong-si Gyenoggi-do 18623 (KR)
(72) Inventor: KIM, Ji Yeon, Seoul 03972 (KR); KIM, Bo Hoon, Yongin-si Gyeonggi-do 17000 (KR)
(74) Representative: Plasseraud IP
(86) International application number: PCT/KR2021/011353
(87) International publication number: WO 2022/045760

(57) **Abstract**

The present invention provides a single dosage form of pharmaceutical composition for treatment of hypertension and hyperlipidemia. According to the present invention, stability and uniform dissolution rates of drugs may be ensured by mixing compositions containing desired drugs and formulating the mixture into a single compartment form, whereby a manufacturing process becomes simple while reducing processing costs. Further, a formulation having biological equivalence to conventional single formulations may be obtained.

## Description

### [Technical Field]

The present invention relates to a pharmaceutical composition in a single dosage form for treatment of hypertension and hyperlipidemia.

### [Background Art]

There are a number of patients suffering from both hypertension and hyperlipidemia simultaneously, and therefore, these patients are usually under prescription of a combination of therapeutic drugs for hypertension and hyperlipidemia. Since it is advantageous for patients to receive prescription of a single dosage form of drugs, for example, Caduet^{™} tablet, which is a combination of atorvastatin and amlodipine, is generally prescribed to the patients as a combined formulation for treatment of hypertension and hyperlipidemia.

Olmesartan medoxomil is an excellent angiotensin II receptor blocker (ARB) and is known to be useful as a medicine for treatment or prevention of hypertension and heart diseases. Olmesartan medoxomil is now commercially available under a trade name of Olmetech^{™} tablet.

Amlodipine is a calcium channel blocker (CCB) and is known to be useful as a medicine for treatment or prevention of hypertension and heart disease. Amlodipine is now commercially available under a trade name of Novask^{™}.

On the other hand, rosuvastatin is a HMG-CoA reductase inhibitor, is used for treatment of hypercholesterolemia, hyperlipidproteinemia and atherosclerosis, and is now sold under a trade name of Cresto^{™} tablet.

Olmesartan medoxomil is an angiotensin II receptor blocker and especially useful for renin-dependent hypertension, while amlodipine has sodium diuretic effects as well as calcium channel extension so as to be useful for renin-nondependent hypertension. Therefore, a combination drug of olmesartan medoxomil and amlodipine was developed to treat hypertension regardless of etiology, which is currently sold under a trade name of Sebica^{™} tablet.

Meanwhile, the applicant of the present invention has disclosed a combined formulation including olmesartan medoxomil and rosuvastatin in Korean Patent Application No. 10-2013-0030734. Currently, a combination drug comprising olmesartan medoxomil and rosuvastatin is commercially available with a trade name of Olostar^{™} tablet.

Further, the present applicant has disclosed a combined formulation comprising olmesartan medoxomil, amlodipine and rosuvastatin in Korean Patent Application No. 10-2019-0022739. Currently, a combination drug comprising olmesartan medoxomil, amlodipine and rosuvastatin is commercially available with a trade name of Olomax^{™} tablet.

### [Disclosure]

### [Technical Problem]

As such, the present applicant has developed a single dosage form of combination drug comprising olmesartan medoxomil and rosuvastatin as described in the conventional art, that is, Korean Patent Application NO. 10-2013-0030734, and has found that olmesartan medoxomil and rosuvastatin have mutual interference or interaction and, even if the above combination drug is prepared as a combination formulation with separated compartments each other to prevent the above response, the combination may ensure bioequivalence with separate single formulations only when a specific disintegrant is used.

Meanwhile, in the conventional art (Korean Patent Application No. 10-2019-0022739), the applicant of the present invention has developed a pharmaceutical composition in a single dosage form comprising olmesartan medoxomil, amlodipine and rosuvastatin, in which an olmesartan demoxomil and an amlodipine compartment and a rosuvastatin compartment are separated, respectively, wherein this combination drug satisfies all requirements in relation to drug dissolution rate and bioequivalence.

However, manufacturing a double layer tablet needs an expensive instrument such as a double layer tablet press, entails low productivity of about 80 to 90% yield, and consumes a longer working time by 2 to 3 times as compared to a single layer tablet, thereby increasing production cost while having reduced production efficiency.

Accordingly, unlike prior arts to partition individual ingredients as compartments, the present invention aims to uniformly release olmesartan medoxomil, amlodipine and rosuvastatin in a single compartment even *in vivo* by designing a formulation to involve uniform dissolution rates for each ingredients, respectively. Moreover, the present invention aims to improve processing stages and production efficiency while reducing processing costs.

### [Technical Solution]

The present inventors have designed and tested a variety of formulations in order to develop a combination drug comprising olmesartan medoxomil, amlodipine and rosuvastatin in a non-compartmental single dosage form and, as a result of the study, have reached the present invention.

The present inventors have confirmed in the development of formulation that, in the case where olmesartan medoxomil, amlodipine or a salt thereof and rosuvastatin or a salt thereof are formulated into a single dosage form, even if compositions comprising the above drugs are not formulated while being divided into separate compartments such as a double layer tablet(bilayer tablet), a core-containing tablet (tablet-in-tablet), etc., stability and dissolution rate similar to those of compartmented and formulated drugs may be obtained when at least one of the drugs is pre-processed into granulates or pellets and then is included in a single formulation. Further, it was confirmed that an elution pattern is similar to that of a control drug.

Further, the present inventors have determined that, when compared with a single formulation containing olmesartan medoxomil and amlodipine during *in vitro* comparative dissolution test under specific dissolution conditions, an dissolution rate of olmesartan medoxomil of the formulation according to the present invention should be designed to be higher at unequal levels on the basis of pharmaceutical equivalence, whereby a formulation biologically equivalent to the single formulation containing olmesartan medoxomil and amlodipine can be obtained. In other words, a combined drug composition of the present invention has a higher initial dissolution rate of olmesartan medoxomil than the conventional combination drug containing olmesartan medoxomil and amlodipine in a single dosage form.

The pharmaceutical composition of the present invention may have a single dosage form comprising olmesartan medoxomil, amlodipine or a salt thereof and rosuvastatin or a salt thereof, wherein these drugs are not divided into compartments. Compared to the pharmaceutical composition in Korean Patent Application No. 10-2019-0022739, which has a separate compartment dosage form such as a bilayer tablet, a tablet-in-tablet, a mini-tablet-containing capsule, etc., the present invention does not use a separate compartment means such as the layer, tablet-in-tablet, mini-tablet, etc. Although not adopting such a compartment means, the present invention may advantageously provide a pharmaceutical composition which satisfies desired drug dissolution rate and requirements in relation to biological equivalence compared to a control drug, wherein at least one composition is prepared in the form of granulates or pellets beforehand, and then applied to formulation of a single dosage form so as to allow mechanical separation against other drugs.

Therefore, the present invention may provide a pharmaceutical composition in a single dosage form, which comprises: a composition comprising olmesartan medoxomil; a composition comprising amlodipine or a salt thereof; and a composition comprising rosuvastatin or a salt thereof,

wherein the above compositions are mixed together and formulated the pharmaceutical composition in a single dosage form, and

wherein one or more of the compositions is comprised in the form of granulates or pellets.

The pharmaceutical composition of the present invention may be formulated into a single dosage form with the single compartment in different manners without using any separation compartment means such as layer, Tab-in-Tab, mini-tablet, etc.

Including at least one of: the composition comprising olmesartan medoxomil; the composition comprising amlodipine or a salt thereof; and the composition comprising rosuvastatin or a salt thereof in the form of granulates or pellets may overcome some problems including, for example, a decrease in production yield due to production of layer, Tab-in-Tab or mini-tablet, increase in working time and production costs, or the like.

Designing the formulation with uniform dissolution rates of each ingredients while allowing simple mechanical separation owing to the granulates or pellets without separation compartment may enable olmesartan medoxomil, amlodipine and rosuvastatin to uniformly release in a single compartment even *in vivo.*

The pharmaceutical composition of the present invention may be formulated in various single dosage forms.

With regard to the pharmaceutical composition of the present invention, at least one of: the composition comprising olmesartan medoxomil; the composition comprising amlodipine or a salt thereof; and the composition comprising rosuvastatin or a salt thereof, may be comprised in the form of granulates or pellets.

For example, one, two or three of the compositions comprising the above drugs, respectively, may have the form of granulates or pellets.

In an embodiment of the resent invention, at least the composition comprising olmesartan medoxomil in the form of granulates or pellets may be comprised in the pharmaceutical composition of a single dosage form. Pre-processing the olmesartan medoxomil-containing composition in the form of granulates or pellets may have advantages of previously overcoming non-soluble property of olmesartan medoxomil and eliminating a problem of difficulties in managing the same during formulation.

Alternatively, olmesartan medoxomil may be mixed with amlodipine or a salt thereof and exist in the form of granulates or pellets. That is, according to an embodiment of the present invention, a composition comprising olmesartan medoxomil and amlodipine or a salt thereof in the form of granulates or pellets may be included in the pharmaceutical composition in a single dosage form.

Components not processed into granulates or pellets may be post-mixing components (extra-granular phase or extra-pellet phase) to be mixed in a formulating process.

Specifically, the pharmaceutical composition of the present invention may be in a single dosage form and, for example, may have a dosage form selected from: a single layer tablet; a capsule comprising one or more selected from the group consisting of granulates, pellets and powder; or a granulate formulation.

According to one embodiment, when the pharmaceutical composition may be formulated in a dosage form of a single layer tablet, which includes: a single layer tablet in which granulates comprising olmesartan medoxomil and amlodipine or a salt thereof and granulates comprising rosuvastatin or a salt thereof are mixed together; a single layer tablet in which granulates comprising olmesartan medoxomil and amlodipine or a salt thereof and a composition comprising rosuvastatin or a salt thereof are mixed together; a single layer tablet in which olmesartan medoxomil granulates, a composition comprising amlodipine or a salt thereof and a composition comprising rosuvastatin or a salt thereof are mixed together; a single layer tablet in which olmesartan medoxomil granulates, a composition comprising amlodipine or a salt thereof and granulates comprising rosuvastatin or a salt thereof are mixed together; or a single layer tablet in which olmesartan medoxomil granulates and granulates comprising amlodipine or a salt thereof and rosuvastatin or a salt thereof are mixed together.

In another embodiment, the pharmaceutical composition of the present invention may be formulated in a dosage form of a capsule, which includes: a capsule in which pellets comprising olmesartan medoxomil and amlodipine or a salt thereof, and pellets or powder comprising rosuvastatin or a salt thereof are comprised; a capsule in which granulates comprising olmesartan medoxomil and amlodipine or a salt thereof, and pellets or powder comprising rosuvastatin or a salt thereof are comprised; a capsule in which pellets comprising olmesartan medoxomil and amlodipine or a salt thereof, and granulates comprising rosuvastatin or a salt thereof are comprised; a capsule in which granulates comprising olmesartan medoxomil and amlodipine or a salt thereof, and granulates comprising rosuvastatin or a salt thereof are comprised; or a capsule in which olmesartan medoxomil granulates, and granulates amlodipine or a salt thereof and rosuvastatin or a salt thereof are comprised.

In another embodiment, when the pharmaceutical composition of the present invention may be formulated into a granulate formulation, which includes: a granulate formulation in which granulates comprising olmesartan medoxomil and amlodipine or a salt thereof and granulates comprising rosuvastatin or a salt thereof are comprised; or a granulate formulation in which olmesartan medoxomil granulates and granulates comprising amlodipine or a salt thereof and rosuvastatin or a salt thereof are comprised.

According to an embodiment of the present invention, the pharmaceutical composition may comprise two or more disintegrants selected from the group consisting of pre-gelatinized starch, sodium croscarmellose, crospovidone, calcium carboxymethyl cellulose, sodium starch glycolate, copovidone and combined silicate.

Although not limited thereto, the disintegrant may be comprised in an amount of 10 to 60 parts by weight ("wt. parts") with regard to total 100 wt. parts of the pharmaceutical composition according to the present invention. Specifically, with regard to total 100 wt. parts of the pharmaceutical composition, 4 to 40 wt. parts of one or more disintegrants selected from pre-gelatinized starch and sodium starch glycolate, as well as 2 to 30 wt. parts of one or more disintegrants selected from the group consisting of sodium croscarmellose, crospovidone, copovidone, low-substituted hydroxylpropyl cellulose and calcium carboxymethyl cellulose may be comprised.

In addition, the pharmaceutical composition may further comprise calcium hydrophosphate hydrate, anhydrous calcium phosphate, calcium carbonate or mixtures thereof. According to the following examples, it was confirmed that formulation stability could be secured when calcium hydrophosphate hydrate, anhydrous calcium phosphate, calcium carbonate or a mixture thereof is comprised in the pharmaceutical composition.

In an embodiment, with regard to total 100 wt. parts of the pharmaceutical composition, calcium hydrophosphate hydrate, anhydrous calcium phosphate, calcium carbonate or a mixture thereof may be included in an amount of 3 to 20 wt. parts.

With regard to the composition comprising olmesartan medoxomil; the composition comprising amlodipine or a salt thereof; the composition comprising olmesartan medoxomil and amlodipine or a salt thereof; the composition comprising rosuvastatin or a salt thereof; or the composition comprising olmesartan medoxomil, amlodipine or a salt thereof, rosuvastatin or a salt thereof, the "composition" may further comprise excipients, disintegrants, additives, etc., other than the drug.

Examples of the excipient may include lactose (including hydrates), dextrin, mannitol, sorbitol, starch, microcrystalline cellulose [e.g., Celphere^{™})], silicified microcrystalline cellulose [e.g., Prosolv^{™})], calcium phosphate hydrate, anhydrous calcium phosphate, calcium carbonate, sugars, or mixtures thereof.

Other additives may include, for example, binders, lubricants, colorants, and the like. The binder may include polyvinylpyrrolidone, povidone, gelatin, starch, sucrose, methyl cellulose, ethyl cellulose, hydroxyethyl cellulose, hydroxypropyl cellulose, hydroxypropylalkyl cellulose (e.g., hydroxypropylmethyl cellulose), and mixtures thereof. The lubricant may include, for example, stearic acid, stearic acid salts (for example, magnesium stearate), talc, corn starch, carnauba wax, hardened anhydrous silicic acid, magnesium silicate, synthetic aluminum silicate, hydrogenated oil, platinum, titanium oxide, microcrystalline cellulose, macrogol 4000 and 6000, isopropyl myristate, calcium hydrophosphate, and mixtures thereof.

Further, in an embodiment, the single layer tablet type pharmaceutical composition of the present invention may be produced by preparing olmesartan medoxomil/amlodipine besylate granulates and rosuvastatin granulates separately, mixing the same, and then, compressing the mixture using a tablet press. The olmesartan medoxomil/amlodipine besylate granulates and rosuvastatin granulates may be prepared through dry granulation or wet granulation according to any conventional process.

In another embodiment, the single layer tablet type pharmaceutical composition of the present invention may be produced by, after preparing olmesartan medoxomil granulates, preparing an amlodipine besylate part and a rosuvastatin calcium salt part, respectively, according to any conventional process, mixing the same, and then, compressing the mixture using a tablet press.

In another embodiment, the single layer tablet type pharmaceutical composition of the present invention may be produced by preparing olmesartan medoxomil granulates and rosuvastatin calcium granulates, respectively, by any conventional process, mixing the same with an amlodipine besylate part, and then, compressing the mixture using a tablet press.

In another embodiment, a single layer tablet type pharmaceutical composition of the present invention may be produced by preparing an olmesartan medoxomil/amlodipine besylate granlulates and a rosuvastatin calcium salt part, respectively, by any conventional process, mixing the same, and then, compressing the mixture using a tablet press.

The pharmaceutical composition of the present invention may be coated with a coating agent, if required, in the case where the composition has a tablet dosage form. According to an embodiment, the pharmaceutical composition may be a single layer tablet coated with a coating agent. The coating agent may further include a typical polymer such as Opadry^{™}. The film coating agent used herein may specifically include polyvinylalcohol (PVA), polyvinylalcohol copolymer, hydroxypropyl methylcellulose (HPMC), etc. An example the polyvinylalcohol copolymer may include, but not limited to, PVA/macrogol grafted polymer. In an embodiment, the pharmaceutical composition of the present invention may be a single layer tablet coated with polyvinylalcohol or polyvinylalcohol copolymer. An amount of the film coating agent used herein is preferably a minimum amount with which the optimal formulation size can be provided, without particular limitation thereof.

When the pharmaceutical composition has a dosage form of a capsule, the capsule may contain one or more selected from the group consisting of granulates, pellets and powder. For example, the capsule may be embodied into various forms such as a granulate-containing capsule, a granulate-powder containing capsule, a pellet-containing capsule, a pellet-powder containing capsule, and the like.

In an embodiment, the pharmaceutical composition in a granulate-powder containing capsule type according to the present invention may be produced by preparing olmesartan medoxomil granulates, an amlodipine besylate mixture and a rosuvastatin mixture, and then filling a capsule with the same.

In another embodiment, the pharmaceutical composition in a granulate-powder containing capsule type according to the present invention may be produced by preparing olmesartan medoxomil/amlodipine besylate granulates and rosuvastatin mixture, and then filling a capsule with the same.

The olmesartan medoxomil granulates, the olmesartan medoxomil/amlodipine besylate granulates and the rosuvastatin granulates may be prepared by any conventional process through dry granulation or wet granulation.

In another embodiment, the pharmaceutical composition in a pellet-powder containing capsule type according to the present invention may be produced by preparing olmesartan medoxomil pellets, an amlodipine besylate mixture and a rosuvastatin mixture, and then filling a capsule with the same.

In another embodiment, the pharmaceutical composition in a pellet-containing capsule form according to the present invention may be produced by preparing olmesartan medoxomil pellets, amlodipine besylate pellets and rosuvastatin pellets, respectively, and then filling a capsule with the same.

In another embodiment, the pharmaceutical composition in a pellet-containing capsule type according to the present invention may be produced by preparing olmesartan medoxomil/amlodipine besylate pellets and rosuvastatin pellets, respectively, and then filling a capsule with the same.

In this regard, the rosuvastatin pellets may be formed by placing beads, for example, non-pareil beads in a fluidized bed coater, dissolving a rosuvastatin calcium salt, an excipient (diluents), a binder and a disintegrant in a suitable solvent, for example, a mixed solvent of water and methanol to prepare a coating solution, and then, coating the beads with the coating solution. A viscosity of the coating solution is preferably in the range of 5 mPa.s to 100 mPa.s. Similarly, the olmesartan medoxomil pellets may also be formed by placing non-pareil beads in a fluidized bed coater, dissolving olmesartan medoxomil, an excipient (diluents) and a binder in a suitable solvent, for example, a mixed solvent of water and methanol to prepare a coating solution, and then, coating the beads with the coating solution.

On the other hand, according to the present invention, the salt of amlodipine may include typical and pharmaceutically acceptable salts, for example, besylate, hydrochloride, hydrobromide, fumarate, citrate, tartrate, maleate, cancylate, lactate, mesylate, camsylate, gluconate, and the like may be used. Preferably, amlodipine besylate is used. On the other hand, the salt of rosuvastatin may include typical and pharmaceutically acceptable salts, for example, calcium salts, hydrochloride, hydrobromide, sulfate, phosphate, acetate, maleate, fumarate, lactate, tartrate, citrate, glconate, besylate, camsylate, and the like may be used. Preferably, the rosuvastatin calcium salt is used.

Although not limited thereto, a particle size of olmesartan medoxomil, amlodipine or a salt thereof, rosuvastatin or a salt thereof may be appropriately adjusted.

When micronization of the drug particle size is required, particles may be crushed using a typical mill, such as a Z-mill, a hammer mill, a ball mill, a fluid energy mill, etc. for micronization of the drug particle. Further, the particle size of the drug can be subdivided by a sieving method using a sieve or a size classification method such as air current classification. Methods for adjusting desired particle sizes are well known in the art. See, for example, the following literature: (Pharmaceutical dosage forms: volume 2, 2nd edition, Ed.: H.A. Lieberman, L. Lachman, J.B. Schwartz (Chapter 3: SIZE REDUCTION)).

In the present specification, the particle size of the drug is expressed with reference to a particle size distribution such as D(X) = Y (where X and Y are positive numbers). In D(X) = Y, Y represents a particle diameter at a point where the particle size of a drug becomes X% (% is calculated based on number, volume or weight) as a result of accumulating the particle sizes in the order of smallest particles, when the particle size distribution of a drug obtained by measuring the particle diameter of the drug in the formulation is expressed on a cumulative curve. For example, D(10) represents the particle diameter at the point where the particle size of the drug becomes 10% as a result of accumulating the particle sizes in the order of smallest particles; D(50) represents the particle size at the point where the particle size of the drug becomes 50% as a result of accumulating the particle sizes in the order of smallest particles; and D(90) represents the particle diameter of the drug at the point where the particle size of the drug becomes 90% as a result of accumulating the particle sizes in the order of smallest particles.

Whether the particle size distribution D(X) represents the percentage of the total cumulative particles by number, volume or weight, may depend on the method used to measure the particle size distribution. Methods of measuring the particle size distribution and types of percentages (%) associated therewith are known in the art. For example, when measuring the particle size distribution by a well-known laser diffraction method, X in D(X) represents the percentage calculated by volume average. It is well known to those skilled in the art that a measured result of the particle size distribution obtained by a specific method may be correlated with those obtained from other techniques based on experience through routine experiments. For example, laser diffraction may provide a volume average particle size in response to a particle volume, which corresponds to a weight average particle size at constant density.

In the present invention, measurement of the particle size distribution of drug particles may be implemented by any commercially available device and the laser diffraction/scattering method based on Mie theory. For example, the measurement is performed using the commercially available device such as a Mastersizer laser diffraction device manufactured by Malvern Instruments. This device is characterized in that, when the particles are irradiated with a helium-neon laser beam and a blue light-emitting diode, scattering occurs and a light scattering pattern appears on a detector, and a particle diameter distribution is obtained by analyzing the above light scattering pattern according to Mie's theory. The measurement method used herein may be either a dry method or a wet method.

Although not limited thereto, in the case of olmesartan medoxomil, D(90) may be 5 to 45 µm, preferably 10 to 30 µm. In the case of amlodipine or a salt thereof, D (90) may be 5 to 100 µm, preferably 10 to 60 µm, more preferably 15 to 45 µm. In the case of rosuvastatin or a salt thereof, D(90) may be 5 to 50 µm, preferably 15 to 40 µm, more preferably 20 to 35 µm. When olmesartan medoxomil, amlodipine or a salt thereof, rosuvastatin or a salt thereof is within the above ranges, respectively, an dissolution rate of the drug at equivalent level and/or an area under curve (AUC) of blood concentration-time and the maximum serum concentration (Cₘₐₓ) at bioequivalent levels as compared to the control drug may be suitably indicated.

The pharmaceutical composition of the present invention may be used for treatment or prevention of hypertension and hyperlipidemia.

The pharmaceutical composition of the present invention may be used for patients who need simultaneous administration of amlodipine, olmesartan medoxomil and rosuvastatin. Currently, the combination drug of olmesartan medoxomil and amlodipine is used for treating essential hypertension that is a disease due to blood pressure not properly controlled with amlodipine or olmesartan medoxomil therapy alone. Further, rosuvastatin is used for treatment of hypercholesterolemia, hyperlipidproteinemia and/or artherosclerosis. Information on application of drugs is already well known.

The present invention may provide a pharmaceutical composition in which an dissolution rate of amlodipine or a salt thereof is equivalent to that of amlodipine or a salt thereof contained in Sebica^{™} tablet. In this regard, whether the dissolution rate represents an equivalent level may be determined according to drug equivalence test management regulations.

Further, the present invention may provide a pharmaceutical composition in which an dissolution rate of rosuvastatin or a salt thereof is equivalent to that of rosuvastatin contained in Cresto^{™} tablet.

Olmesartan medoxomil and amlodipine or a salt thereof in the pharmaceutical composition of the present invention are characterized in that the area under curve (AUC) of blood concentration-time and the maximum serum concentration (Cₘₐₓ) have substantially bioequivalent levels as compared to Sebica^{™} tablet having the same active ingredient dose.

Further, the rosuvastatin calcium salt in the pharmaceutical composition of the present invention represents the area under curve (AUC) of blood concentration-time and the maximum serum concentration (Cₘₐₓ) at substantially bioequivalent levels as compared to Cresto^{™} tablet having the same active ingredient dose.

Accordingly, the pharmaceutical composition of the present invention may be specifically useful for patients who require a combination therapy of amlodipine and olmesartan medoxomil and also need administration of rosuvastatin simultaneously.

With regard to the pharmaceutical composition of the present invention, the active ingredients, that is, olmesartan medoxomil, amlodipine or a salt thereof, and rosuvastatin or a salt thereof may be used in a therapeutically effective amount. The therapeutically effective amount may vary depending on patient's symptoms, age, weight, and severity of diseases.

Although not limited thereto, for example, on the basis of a unit formulation (i.e., unit dosage form), olmesartan medoxomil may be used in an amount of about 5 mg to about 80 mg, preferably about 10 mg to about 40 mg. Further, on the basis of the unit formulation (i.e., unit dosage form), amlodipine or a salt thereof may be used in an amount of about 2.5 mg to 10 mg. Further, on the basis of the unit formulation (i.e., unit dosage form), rosuvastatin or a salt thereof may be used in an amount of about 2 mg to about 40 mg, preferably about 5 mg to about 20 mg.

According to an embodiment, the pharmaceutical composition of the present invention may comprise 40 mg of olmesartan medoxomil, 10 mg of amlodipine or a salt thereof, and 20 mg of rosuvastatin or a salt thereof.

According to another embodiment, the pharmaceutical composition of the present invention may comprise 40 mg of olmesartan medoxomil, 10 mg of amlodipine or a salt thereof, and 10 mg of rosuvastatin or a salt thereof.

According to another embodiment, the pharmaceutical composition of the present invention may comprise 40 mg of olmesartan medoxomil, 5 mg of amlodipine or a salt thereof, and 10 mg of rosuvastatin or a salt thereof.

According to another embodiment, the pharmaceutical composition of the present invention may comprise 40 mg of olmesartan medoxomil, 5 mg of amlodipine or a salt thereof, and 5 mg of rosuvastatin or a salt thereof.

According to another embodiment, the pharmaceutical composition of the present invention may comprise 20 mg of olmesartan medoxomil, 5 mg of amlodipine or a salt thereof, and 10 mg of rosuvastatin or a salt thereof.

According to another embodiment, the pharmaceutical composition of the present invention may comprise 20 mg of olmesartan medoxomil, 5 mg of amlodipine or a salt thereof, and 5 mg of rosuvastatin or a salt thereof.

According to another embodiment, the pharmaceutical composition of the present invention may comprise 20 mg of olmesartan medoxomil, 5 mg of amlodipine or a salt thereof, and 2.5 mg of rosuvastatin or a salt thereof.

According to an embodiment, the pharmaceutical composition of the present invention may comprise 40 mg of olmesartan medoxomil, 2.5 mg of amlodipine or a salt thereof, and 20 mg of rosuvastatin or a salt thereof.

According to another embodiment, the pharmaceutical composition of the present invention may comprise 40 mg of olmesartan medoxomil, 2.5 mg of amlodipine or a salt thereof, and 10 mg of rosuvastatin or a salt thereof.

According to another embodiment, the pharmaceutical composition of the present invention may comprise 20 mg of olmesartan medoxomil, 2.5 mg of amlodipine or a salt thereof, and 10 mg of rosuvastatin or a salt thereof.

According to another embodiment, the pharmaceutical composition of the present invention may comprise 20 mg of olmesartan medoxomil, 2.5 mg of amlodipine or a salt thereof, and 5 mg of rosuvastatin or a salt thereof.

According to another embodiment, the pharmaceutical composition of the present invention may comprise 20 mg of olmesartan medoxomil, 5 mg of amlodipine or a salt thereof, and 2.5 mg of rosuvastatin or a salt thereof.

The pharmaceutical composition according to the present invention may be administered once a day, but it is not limited thereto.

### [Advantageous effects]

As described above, according to the present invention, stability and uniform dissolution rate may be ensured by mixing the compositions containing drugs and then formulating the mixture in a single compartment form, whereby a simple manufacturing process can be achieved while reducing processing cost, and a formulation having biological equivalence can be obtained as compared to conventional single dosage formulations.

### [Description of Drawings]

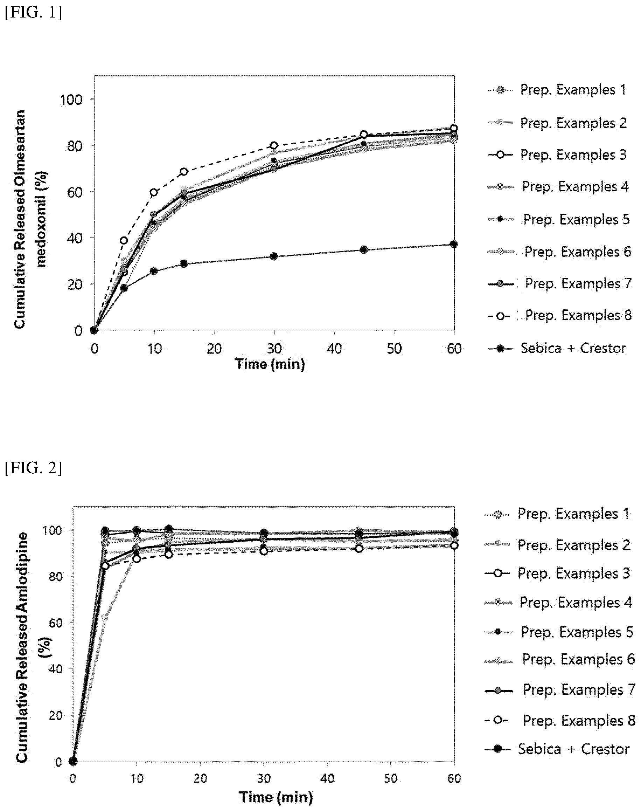

FIG. 1 illustrates the results of dissolution tests of formulations in Preparative Examples 1 to 8 compared to a control drug with regard to olmesartan medoxomil.
FIG. 2 illustrates the results of dissolution tests of formulations in Preparative Examples 1 to 8 compared to a control drug with regard to amlodipine besylate.

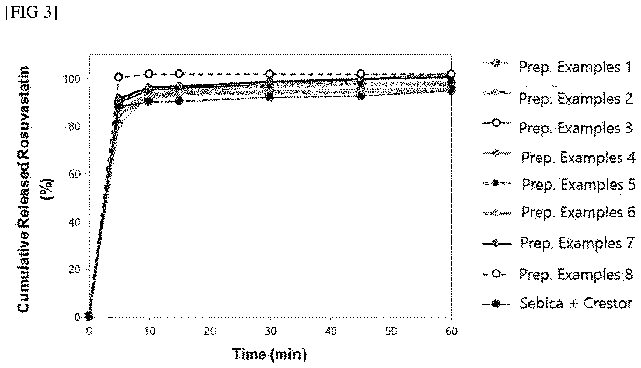

FIG. 3 illustrates the results of dissolution rates of formulations in Preparative Examples 1 to 8 compared to a control drug with regard to rosuvastatin calcium salt.

### [Best Mode]

### [Example]

Hereinafter, the present invention will be described in more detail in the following examples. However, the following examples illustrate only the content of the present invention and are not intended to limit or restrict the scope of the present invention resent invention. What is easily inferred by those skilled in the art from the detailed description and examples of the present invention is construed as belonging to the scope of the present invention.

### [Preparation Example]

### Preparative Example 1 to 6: Preparation of single layer tablet comprising olmesartan/amlodipine composition and rosuvastatin composition

A formulation of each of the preparative examples was prepared with change in terms of types of additives such as disintegrants, excipients, etc. and content thereof.

**[TABLE 1]**

| **Ingredient** | | **Prep. Example 1** | **Prep. Example 2** | **Prep. Example 3** | **Prep. Example 4** | **Prep. Example 5** | **Prep. Example 6** |
|---|---|---|---|---|---|---|---|
| | Olmesartan medoxomil | 40 | 40 | 40 | 40 | 40 | 40 |
| Composi tion 1 | Amlodipine besylate | 13.89 | 13.89 | 13.89 | 13.89 | 13.89 | 13.89 |
| | Microcrystalline cellulose | - | - | - | 66.11 | - | 38.11 |
| | Silicified microcrystalline cellulose | 76.11 | 90.11 | 91.11 | - | 69.11 | - |
| | Lactose hydrate | - | - | - | - | - | 20 |
| | Hydroxypropyl cellulose | - | - | - | - | - | 5 |
| | Povidone | - | - | - | - | 7 | - |
| | Hydroxypropyl cellulose methyl cellulose | - | - | 5 | - | - | - |
| | Pre-gelatinized starch | 35 | - | 15 | 35 | 35 | - |
| | Sodium starch glycolate | - | 21 | - | - | - | 35 |
| | Sodium croscarmellose | 15 | 15 | 5 | 5 | - | - |
| | Crospovidone | - | - | 10 | - | - | - |
| | Copovidione | - | - | - | - | 15 | - |
| | Calcium carboxymethyl cellulose | - | - | - | 20 | - | - |
| | Low-substituted hydroxypropyl cellulose | - | - | - | - | - | 28 |
| Composi tion 2 | Rosuvastatin calcium | 10.4 | 10.4 | 10.4 | 10.4 | 10.4 | 10.4 |
| | Lactose hydrate | 79.8 | 42.6 | 65.6 | 74.8 | 41.8 | 74.8 |
| | Microcrystalline cellulose | - | 30 | - | - | 25.8 | - |
| | Silicified microcrystalline cellulose | 41.8 | - | 40 | 15.8 | - | 45 |
| | Calcium hydrophosphate hydrate | 18 | - | - | 36 | 54 | - |
| | Anhydrous calcium phosphate | - | - | 36 | - | - | - |
| | Calcium carbonate | - | 54 | - | - | - | 21.8 |
| | Sodium starch glycolate | - | - | - | 15 | 5 | - |
| | Crospovidone | 15 | 10 | - | - | - | 15 |
| | Sodium croscarmellose | 7 | 5 | - | - | - | 5 |
| | Low-substituted hydroxypropyl cellulose | - | 20 | - | 20 | 35 | - |
| | Combined silicate | - | - | 20 | - | - | - |
| Post-mixing | Colloidal silicon dioxide | 4 | 4 | 4 | 4 | 4 | 4 |
| | Magnesium stearate | 4 | 4 | 4 | 4 | 4 | 4 |
| Sum | Total mass per tablet | 360 | 360 | 360 | 360 | 360 | 360 |

In Preparative Examples 1 to 6, after forming granulates comprising olmesartan medoxomil and amlodipine besylate as well as rosuvastatin calcium granulates, these granulates were mixed with lubricants (post-mixing), followed by compressing the same to prepare single layer tablets.

**[TABLE 2]**

| **Ingredient** | | **Prep. Example 7** | **Prep. Example 8** |
|---|---|---|---|
| Compositi on 1 | Olmesartan medoxomil | 40 | 40 |
| | Silicified microcrystalline cellulose | 76.11 | 45.11 |
| | Calcium hydrophosphate hydrate | - | 36 |
| | Pre-gelatinized starch | 35 | 35 |
| | Sodium croscarmellose | 10 | - |
| | Crospovidone | 5 | 10 |
| Post-mixing 1 | Amlodipine besylate | 13.89 | 13.89 |
| | Rosuvastatin calcium | 10.4 | 10.4 |
| | Lactose hydrate | 76.8 | 63.93 |
| | Silicified microcrystalline cellulose | 38 | 41.67 |
| | Calcium hydrophosphate hydrate | 21.8 | - |
| | Calcium carbonate | - | 36 |
| | Sodium starch glycolate | 25 | - |
| | Crospovidone | - | 10 |
| | Sodium croscarmellose | - | 10 |
| Post-mixing 2 | Colloidal silicon dioxide | 4 | 4 |
| | Magnesium stearate | 4 | 4 |
| Sum | Total mass per tablet | 360 | 360 |

In Preparative Examples 7 and 8, after forming granulates comprising olmesartan medoxomil, the formed granulates were mixed with a composition comprising amlodipine and rosuvastatin calcium (post-mixing 1) and lubricants (post-mixing 2), followed by compressing the same to prepare single layer tablets.

### [Experimental Example]

### Experimental Example 1: Dissolution test

Comparative dissolution tests were performed on the formulations prepared in the preparative examples of the present invention under the following dissolution test conditions.

As a control drug used in the comparative dissolution tests, 10/40 mg of Sebica^{™} tablets (amlodipine besylate/olmesartan medoxomil) and 20 mg of Cresto^{™} tablets (rosuvastatin calcium) were used.

Sebica^{™} and Cresto^{™} tablets have each film-coated single tablet form. Sebica^{™} tablet comprises silicified microcrystalline cellulose, pre-gelatinized starch, sodium croscarmellose and magnesium stearate as excipients, and further comprises polyvinylalcohol, macrogol/polyethyleneglycol 3350, titanium dioxide, talc and iron oxide as film coating agents.

On the other hand, Cresto^{™} tablet comprises microcrystalline cellulose, lactose hydrate, tribasic calcium phosphate, crospovidone and magnesium as excipients, and comprises hypromellose, triacetin, titanium dioxide and iron oxide as film coating agents.

### [Dissolution test conditions]

Dissolution solution: solution 1 or solution 2 in Korean Pharmacopoeia Disintegration Test Method, or water
Omesartan medoxomil: Water
Amlodipine besylate: solution 1, pH 1.2
Rosuvastatin calcium: solution 2, pH 6.8
Temperature: 37 ± 0.5 °C
Test method: Korean Pharmacopoeia dissolution test method No. 2 (paddle method)
Paddle rotation speed: 50 rpm
Analysis method: UPLC method
(^{∗}) UPLC operating conditions
□ Column: ACQUITY UPLC BEH C18 (2.1 × 50 mm 1.7 µm)
□ Detector: Ultraviolet absorbance photometer (239 nm)
□ Flow rate: 0.1 mL/min
□ Analysis time: 30 minutes
□ Mobile phase: Phosphoric acid buffer/acetonitrile = 80/20

The dissolution test results of the above formulations are shown in the table below.

**[TABLE 3] Olmesartan medoxomil dissolution rate**

| **Dissolution time** | **0 min** | **5 min** | **10 min** | **15 min** | **30 min** | **45 min** | **60 min** |
|---|---|---|---|---|---|---|---|
| Prep. Example 1 | 0 | 18.18 | 44.36 | 54.91 | 71.05 | 78.42 | 82.22 |
| Prep. Example 2 | 0 | 29.82 | 49.87 | 60.65 | 76.75 | 83.80 | 87.84 |
| Prep. Example 3 | 0 | 24.87 | 44.31 | 55.82 | 72.13 | 79.65 | 83.42 |
| Prep. Example 4 | 0 | 26.46 | 45.55 | 56.53 | 72.72 | 80.58 | 84.59 |
| Prep. Example 5 | 0 | 27.12 | 46.27 | 57.20 | 73.04 | 80.24 | 83.33 |
| Prep. Example 6 | 0 | 26.62 | 43.94 | 54.59 | 70.09 | 78.10 | 81.89 |
| Prep. Example 7 | 0 | 25.98 | 49.96 | 59.26 | 69.48 | 83.96 | 85.30 |
| Prep. Example 8 | 0 | 38.80 | 59.46 | 68.63 | 79.86 | 84.72 | 87.37 |
| Sebica+Cresto | 0 | 18.17 | 25.43 | 28.61 | 31.79 | 34.83 | 37.08 |

**[TABLE 4] Amlodipine besylate dissolution rate**

| **Dissolution time** | **0 min** | **5 min** | **10 min** | **15 min** | **30 min** | **45 min** | **60 min** |
|---|---|---|---|---|---|---|---|
| Prep. Example 1 | 0 | 94.25 | 95.83 | 96.35 | 95.79 | 95.02 | 95.03 |
| Prep. Example 2 | 0 | 61.86 | 90.33 | 94.85 | 96.22 | 95.19 | 96.02 |
| Prep. Example 3 | 0 | 97.98 | 99.65 | 98.71 | 98.30 | 98.54 | 98.91 |
| Prep. Example 4 | 0 | 83.40 | 90.63 | 91.57 | 91.84 | 92.04 | 93.44 |
| Prep. Example 5 | 0 | 90.34 | 90.03 | 91.22 | 92.30 | 92.13 | 92.87 |
| Prep. Example 6 | 0 | 96.83 | 95.11 | 98.30 | 98.44 | 99.79 | 99.25 |
| Prep. Example 7 | 0 | 85.64 | 91.80 | 93.35 | 96.14 | 96.52 | 99.38 |
| Prep. Example 8 | 0 | 84.10 | 87.36 | 89.11 | 90.62 | 91.75 | 93.24 |
| Sebica+Cresto | 0 | 99.67 | 99.77 | 100.37 | 98.57 | 98.48 | 98.53 |

**[TABLE 5] Rosuvastatin calcium dissolution rate**

| **Dissolution time** | **0 min** | **5 min** | **10 min** | **15 min** | **30 min** | **45 min** | **60 min** |
|---|---|---|---|---|---|---|---|
| Prep. Example 1 | 0 | 80.67 | 92.55 | 93.91 | 94.69 | 95.48 | 95.82 |
| Prep. Example 2 | 0 | 90.45 | 95.87 | 96.43 | 96.66 | 97.29 | 97.16 |
| Prep. Example 3 | 0 | 89.75 | 94.86 | 95.89 | 97.36 | 97.90 | 98.05 |
| Prep. Example 4 | 0 | 84.60 | 91.66 | 93.61 | 97.26 | 99.54 | 101.71 |
| Prep. Example 5 | 0 | 86.82 | 93.26 | 94.79 | 96.71 | 97.67 | 98.43 |
| Prep. Example 6 | 0 | 87.55 | 91.74 | 93.37 | 93.84 | 94.15 | 94.56 |
| Prep. Example 7 | 0 | 91.52 | 96.12 | 96.65 | 98.51 | 99.82 | 100.41 |
| Prep. Example 8 | 0 | 100.51 | 101.74 | 101.91 | 101.8 | 101.81 | 101.78 |
| Sebica+Cresto | 0 | 87.92 | 89.97 | 90.37 | 91.80 | 92.50 | 94.66 |

All of the formulations in Preparation Examples 1 to 6 had higher dissolution rates for olmesartan compared to the control drug at all times, while the dissolution rates for amlodipine and rosuvastatin, respectively, were not significantly different from each other, that is, amlodipine and rosuvastatin showed similar dissolution rates compared to the control drug. In fact, although the constitutional composition of olmesartan/amlodipine slightly affected initial dissolution rate of rosuvastatin, it was confirmed that there is no significant influence on overall dissolution profile of rosuvastatin.

In general, for determination of similarity in the comparative dissolution test, the Ministry of Food and Drug Safety Notice No. 2017-28, Chapter 3, Article 21 was referred. In the case of olmesartan, since the average dissolution rate of the control drug is less than 50% within the defined test time, the dissolution rate deviation must be within ±8% or the similarity factor value (f2) should be 55 or more, so as to be determined to have equivalence. In the formulations of Preparation Examples 1 to 8, it could be seen that the dissolution rate of olmesartan is not equivalent to that of the control drug, and has high deviation of 30% or more relative to the control drug. In the case of amlodipine and rosuvastatin, the average dissolution rate of the control drug is 85% or more within 15 minutes so that, if the dissolution rate deviation is within ±15%, it may be determined as equivalent.

### Preparative Example 1A: Preparation of coating tablets

The naked tablet prepared in Preparative Example 1 was coated with the coating agent according to the following constitutional compositions, thereby forming a coating tablet of Preparative Example 1A.

**[TABLE 6] Constitutional composition of single layer coating tablet comprising olmesartan/amlodipine composition and rosuvastatin composition**

| **Ingredient** | | **Prep. Example 1A** |
|---|---|---|
| Composition 1 | Olmesartan medoxomil | 40 |
| | Amlodipine besylate | 13.89 |
| | Silicified microcrystalline cellulose | 76.11 |
| | Pre-gelatinized starch | 35 |
| | Sodium croscarmellose | 15 |
| Composition 2 | Rosuvastatin calcium | 10.4 |
| | Lactose hydrate | 79.8 |
| | Silicified microcrystalline cellulose | 38 |
| | Calcium hydrophosphate hydrate | 21.8 |
| | Crospovidone | 15 |
| | Sodium croscarmellose | 7 |
| Post-mixing | Colloidal silicon dioxide | 4 |
| | Magnesium stearate | 4 |
| Sum | Total mass per tablet | 360 |
| Coating | Opadry^{™} | 12.00 |
| **Total mass per tablet (coating tablet)** | | **372.00** |

The dissolution test results of the formulation in Preparative Example 1A were similar to that of the formulation in Preparative Example 1.

### Preparative Example 9: Preparation of coating tablets

Coating tablets in Preparative Examples 9 to 13 were prepared according to the following constitutional compositions.

**[TABLE 7] Constitutional composition of single layer coating tablets comprising olmesartan/amlodipine/rosuvastatin compositions**

| **Ingredient** | | **Prep. Example 9** | **Prep. Example 10** | **Prep. Example 11** | **Prep. Example 12** | **Prep. Example 13** |
|---|---|---|---|---|---|---|
| Compositi on | Olmesartan medoxomil | 40 | 40 | 40 | 40 | 40 |
| | Amlodipine besylate | 13.89 | 13.89 | 13.89 | 13.89 | 13.89 |
| | Rosuvastatin calcium | 10.4 | 10.4 | 10.4 | 10.4 | 10.4 |
| | Lactose hydrate | 46.71 | 79.8 | 35.71 | 82.71 | 48.71 |
| | Microcrystalline cellulose | 102 | - | 58 | 102 | 70 |
| | Silicified microcrystalline cellulose | 50 | 117.91 | - | 50 | - |
| | Pre-gelatinized starch | 35 | 35 | 150 | 35 | 35 |
| | Crospovidone | - | 15 | - | - | - |
| | Sodium croscarmellose | 26 | 22 | 26 | - | 26 |
| | Calcium hyhdrophosphate hydrate | - | 18 | 18 | 18 | - |
| | Calcium carbonate | - | - | - | - | 108 |
| | Colloidal silicon dioxide | 4 | 4 | 4 | 4 | 4 |
| | Magnesium stearate | 4 | 4 | 4 | 4 | 4 |
| Coating | Opadry^{™} | 12.00 | 12.00 | 12.00 | 12.00 | 12.00 |
| **Total mass per tablet (coating tablet)** | | 372.00 | 372.00 | 372.00 | 372.00 | 372.00 |

In Preparative Examples 9 to 13, the compositions were mixed/compressed to produce single layer tablets, followed by coating the same without pre-preparation of granulates or pellets. The results of the dissolution rate tests for the formulations in Preparative Examples 9 to 13 were different from the results of the same tests for the formulations in Preparative Example 1 to 8. Although the initial dissolution rate of olmesartan medoxomil within 15 minutes was substantially equivalent to that of the control drug, thereafter, it was confirmed that the dissolution rate was increased to a non-equivalent level. In preparative Examples 9 to 13, amlodipine besylate and rosuvastatin calcium showed each initial dissolution rate lower than that of the control drug, thereby demonstrating non-equivalence.

**[TABLE 8] Olmesartan medoxomil dissolution rate**

| **Dissolution time** | **0 min** | **5 min** | **10 min** | **15 min** | **30 min** | **45 min** | **60 min** |
|---|---|---|---|---|---|---|---|
| Prep. Example 9 | 0 | 18.37 | 26.63 | 33.65 | 41.02 | 50.42 | 58.44 |
| Prep. Example 10 | 0 | 18.37 | 30.63 | 37.65 | 47.02 | 51.42 | 54.43 |
| Prep. Example 11 | 0 | 17.71 | 37.42 | 43.21 | 55.77 | 60.18 | 65.02 |
| Prep. Example 12 | 0 | 13.21 | 25.33 | 40.16 | 49.93 | 57.03 | 63.89 |
| Prep. Example 13 | 0 | 13.08 | 24.17 | 39.21 | 50.33 | 55.02 | 58.96 |
| Sebica+Cresto | 0 | 18.17 | 25.43 | 28.61 | 31.79 | 34.83 | 37.08 |

**[TABLE 9] Amlodipine besylate dissolution rate**

| **Dissolution time** | **0 min** | **5 min** | **10 min** | **15 min** | **30 min** | **45 min** | **60 min** |
|---|---|---|---|---|---|---|---|
| Prep. Example 9 | 0 | 64.57 | 72.33 | 85.51 | 88.97 | 90.28 | 92.31 |
| Prep. Example 10 | 0 | 84.34 | 95.05 | 96.34 | 97.89 | 98.91 | 99.57 |
| Prep. Example 11 | 0 | 56.73 | 87.37 | 90.01 | 92.17 | 93.39 | 94.41 |
| Prep. Example 12 | 0 | 55.59 | 73.89 | 84.19 | 90.06 | 91.76 | 93.59 |
| Prep. Example 13 | 0 | 43.84 | 89.08 | 90.33 | 91.97 | 92.94 | 94.09 |
| Sebica+Cresto | 0 | 99.67 | 99.77 | 100.37 | 98.57 | 98.48 | 98.53 |

**[TABLE 10] Rosuvastatin calcium dissolution rate**

| **Dissolution time** | **0 min** | **5 min** | **10 min** | **15 min** | **30 min** | **45 min** | **60 min** |
|---|---|---|---|---|---|---|---|
| Prep. Example 9 | 0 | 75.63 | 87.74 | 92.81 | 93.79 | 94.84 | 95.29 |
| Prep. Example 10 | 0 | 88.91 | 90.03 | 93.89 | 94.44 | 95.81 | 96.63 |
| Prep. Example 11 | 0 | 57.24 | 68.91 | 82.37 | 89.01 | 91.14 | 92.24 |
| Prep. Example 12 | 0 | 82.67 | 90.45 | 93.33 | 94.02 | 94.89 | 95.71 |
| Prep. Example 13 | 0 | 63.37 | 80.46 | 88.21 | 92.48 | 93.39 | 95.52 |
| Sebica+Cresto | 0 | 87.92 | 89.97 | 90.37 | 91.80 | 92.50 | 94.66 |

### Experimental Example 2: Stability test

With regard to the formulations of Preparative Examples 1 to 10, each formulation was coated with Opadry^{™}, packed in a bottle (including silica gel) and stored under stress testing conditions until a chemical stability test between major ingredients and excipients was conducted. More particularly, a HDPE bottle in which tablets are placed was stored under stress testing conditions (50 °C, 80% relative humidity) for 4 weeks, followed by confirming total content of related substance (%) through UPLC.

### ^{∗} UPLC operating conditions

□ Column: ACQUITY UPLC BEH C18 (2.1 × 50 mm 1.7 µm)
□ Detector: Ultraviolet absorbance photometer (239 nm)
□ Flow rate: 0.1 mL/min
□ Analysis time: 30 minutes
□ Mobile phase: Phosphoric acid buffer/acetonitrile = 80/20

**[TABLE 11] Stability test result**

| | Item | Initial | at 2 week under stress testing | at 4 week under stress testing |
|---|---|---|---|---|
| Prep. Example 1 | Olmesartan-derived related substance | 0.63 | 0.74 | 0.78 |
| | Amlodipine-derived related substance | 0.09 | 0.09 | 0.09 |
| | Rosuvastatin-derived related substance | 0.29 | 0.41 | 0.85 |
| Prep. Example 2 | Olmesartan-derived related substance | 0.51 | 0.63 | 0.72 |
| | Amlodipine-derived related substance | 0.00 | 0.00 | 0.00 |
| | Rosuvastatin-derived related substance | 0.25 | 0.45 | 0.68 |
| Prep. Example 3 | Olmesartan-derived related substance | 0.64 | 0.76 | 0.81 |
| | Amlodipine-derived related substance | 0.00 | 0.00 | 0.00 |
| | Rosuvastatin-derived related substance | 0.11 | 0.34 | 0.77 |
| Prep. Example 4 | Olmesartan-derived related substance | 0.48 | 0.53 | 0.61 |
| | Amlodipine-derived related substance | 0.00 | 0.00 | 0.00 |
| | Rosuvastatin-derived related substance | 0.34 | 0.52 | 0.78 |
| Prep. Example 5 | Olmesartan-derived related substance | 0.55 | 0.64 | 0.77 |
| | Amlodipine-derived related substance | 0.00 | 0.00 | 0.00 |
| | Rosuvastatin-derived related substance | 0.21 | 0.43 | 0.65 |
| Prep. Example 6 | Olmesartan-derived related substance | 0.41 | 0.55 | 0.62 |
| | Amlodipine-derived related substance | 0.00 | 0.00 | 0.00 |
| | Rosuvastatin-derived related substance | 0.18 | 0.36 | 0.54 |
| Prep. Example 7 | Olmesartan-derived related substance | 0.55 | 0.72 | 0.88 |
| | Amlodipine-derived related substance | 0.00 | 0.00 | 0.00 |
| | Rosuvastatin-derived related substance | 0.66 | 0.83 | 0.98 |
| Prep. Example 8 | Olmesartan-derived related substance | 0.81 | 0.96 | 1.11 |
| | Amlodipine-derived related substance | 0.10 | 0.13 | 0.17 |
| | Rosuvastatin-derived related substance | 0.55 | 0.66 | 0.78 |
| Prep. Example 9 | Olmesartan-derived related substance | 0.42 | 0.96 | 1.11 |
| | Amlodipine-derived related substance | 0.18 | 0.36 | 0.56 |
| | Rosubastatin-derived related substance | 0.75 | 1.66 | 2.78 |
| Prep. Example 10 | Olmesartan-derived related substance | 0.61 | 0.75 | 0.97 |
| | Amlodipine-derived related substance | 0.00 | 0.01 | 0.01 |
| | Rosuvastatin-derived related substance | 0.23 | 0.93 | 1.91 |

All of the formulations in Preparative Examples 1 to 8 did not have a significant difference in terms of contents of related substances, thereby confirming effectively maintained stability. On the other hand, in the case of Preparative Example 9 in which calcium hydrophosphate or calcium carbonate is not included and separate drugs are simply mixed to prepare the formulation, it was confirmed that the related substance is remarkably increased after 4 weeks under stress testing conditions to show deteriorated stability. Based on the results of Preparative Examples 3, 4 and 6 to 9, it was confirmed that calcium hydrophosphate, anhydrous calcium phosphate and calcium carbonate are excipients having similar influence on stability of each component.

### Experimental Example 3: Bioequivalence test

The formulation of Preparation Example 1 was subjected to a pharmacokinetic test (PK test) to evaluate its bioequivalence with the control drug.

That is, 36 beagle dogs were divided into 2 groups of 16 each, wherein a first group was orally administered with the tablets of Preparation Example 1 while a second group received oral administration of 10/40 mg of Sebica tablets (amlodipine besylate/olmesartan medoxomil) and 10 mg of Cresto tablets in combination. Blood was collected at 0, 0.5, 1, 1.5, 2, 2.5, 3, 4, 5, 6, 8, 10, 12, 16, 24, 48 and 72 hours after administration, and UPLC-MS/MS (Waters ACQUITY UPLCTM system) was used to quantify the blood concentrations of olmesartan, amlodipine and rosuvastatin, respectively. After quantification, AUC and Cₘₐₓ of each of olmesartan, amlodipine and rosuvastatin at the time of administration of the control drug (combined administration) and the test drug were statistically processed to evaluate bioequivalence between the formulations.

The bioequivalence evaluation was conducted in accordance with the standard guidelines for bioequivalence of the KFDA. That is, after log transformation of the AUC and Cₘₐₓ values of olmesartan, amlodipine and rosuvastatin, the geometric mean was estimated, followed by calculating projected 90% confidence intervals for geometric mean ratio. If the 90% confidence interval ranges from 0.8 to 1.25, two formulations under comparison are considered to be biologically equivalent.

Results of the bioequivalence evaluation conducted as described above are shown in **Table 12** below. In **Table 12**, T/R ratio was obtained by dividing the geometric mean of the evaluation items for test formulations by the geometric mean of the evaluation items for a control formulation [i.e., T/R ratio = geometric mean of evaluation items (test formulation)/geometric mean of evaluation items (control)]. If the T/R ratio is higher than 1, it means that the test drug is averagely absorbed more than the control drug, or that the maximum serum concentration of the test drug is averagely higher than the control drug. On the contrary, if T/R ratio is less than 1, it means that the test drug is averagely less absorbed than the control group, or that the blood concentration of the test drug is averagely lower than that of the control drug. In other words, as the T/R ratio is getting farther from 1, it is determined with high probability to be biologically unequal.

**[TABLE 12]**

| **PK of single layer tablets of Preparative Example 1** | | |
|---|---|---|
| **Ingredient** | **PK parameter** | **90% confidence interval (T/R ratio)** |
| Olmesartan | AUC | 0.9669 - 1.1171 (1.039) |
| | Cmax | 0.8598 - 1.0699 (0.959) |
| Amlodipine | AUC | 0.9144 - 1.0028 (0.957) |
| | Cmax | 0.9243 - 0.9895 (0.956) |
| Rosuvastatin | AUC | 0.8842 - 1.1975 (1.028) |
| | Cmax | 0.8622 - 1.2224 (1.067) |

### Preparative Examples 14 to 19: Preparation of low dose single layer tablets

Single layer tablets of Preparative Examples 14 to 16 were prepared with the following constitutional compositions on the basis of the constitutional compositions of the single layer tablets of Preparative Example 1.

**[TABLE 13]**

| **Constitutional composition of single layer tablets comprising olmesartan/amlodipine composition and rosuvastatin composition** | | | | | | | |
|---|---|---|---|---|---|---|---|
| **Ingredient** | | **Prep. Example 14** | **Prep. Example 15** | **Prep. Example 16** | **Prep. Example 17** | **Prep. Example 18** | **Prep. Example 19** |
| Composi tion 1 | Olmesartan medoxomil | 40 | 40 | 40 | 20 | 20 | 20 |
| | Amlodipine besylate | 13.89 | 6.945 | 6.945 | 6.945 | 6.945 | 6.945 |
| | Silicified microcrystalline cellulose | 76.11 | 83.055 | 83.055 | 38.055 | 38.055 | 38.055 |
| | Pre-gelatinized starch | 35 | 35 | 35 | 17.5 | 17.5 | 17.5 |
| | Sodium croscarmellose | 15 | 15 | 15 | 7.5 | 7.5 | 7.5 |
| Composi tion 2 | Rosuvastatin calcium | 5.2 | 10.4 | 5.2 | 10.4 | 5.2 | 2.6 |
| | Lactose hydrate | 85 | 79.8 | 85 | 34.7 | 39.9 | 42.5 |
| | Silicified microcrystalline cellulose | 38 | 38 | 38 | 19 | 19 | 19 |
| | Calcium hydrophosphate hydrate | 21.8 | 21.8 | 21.8 | 10.9 | 10.9 | 10.9 |
| | Crospovidone | 15 | 15 | 15 | 7.5 | 7.5 | 7.5 |
| | Sodium croscarmellose | 7 | 7 | 7 | 3.5 | 3.5 | 3.5 |
| Post-mixing | Colloidal silicon dioxide | 4 | 4 | 4 | 2 | 2 | 2 |
| | Magnesium stearate | 4 | 4 | 4 | 2 | 2 | 2 |
| Sum | Total mass per tablet | 360 | 360 | 360 | 180 | 180 | 180 |

In Preparative Examples 14 to 19, granulates comprising olmesartan medoxomil and amlodipine besylate and rosuvastatin calcium granulates were prepared, respectively, and these granulates were mixed with lubtricants (post-mixing), followed by compressing the mixture to form single layer tablets.

Dissolution test results of the formulations in Preparative Examples 14 to 19 are shown in Tables 14 to 16 below.

**[TABLE 14]**

| **Olmesartan medoxomil (pH 6.8)** | | | | | | | |
|---|---|---|---|---|---|---|---|
| **Dissolution time** | **0 min** | **5 min** | **10 min** | **15 min** | **30 min** | **45 min** | **60 min** |
| Prep. Example 14 | 0 | 46.04 | 60.52 | 67.08 | 75.31 | 78.66 | 79.76 |
| Prep. Example 15 | 0 | 34.16 | 51.61 | 58.93 | 67.64 | 71.68 | 74.06 |
| Prep. Example 16 | 0 | 31.30 | 51.54 | 59.26 | 69.22 | 73.45 | 76.35 |
| Prep. Example 17 | 0 | 43.99 | 60.79 | 66.89 | 73.78 | 75.62 | 77.23 |
| Prep. Example 18 | 0 | 45.20 | 59.93 | 65.98 | 73.93 | 76.87 | 79.39 |
| Prep. Example 19 | 0 | 44.15 | 57.36 | 62.74 | 71.80 | 75.32 | 77.13 |

**[TABLE 15]**

| **Amlodipine besylate (pH 1.2)** | | | | | | | |
|---|---|---|---|---|---|---|---|
| **Dissolution time** | **0 min** | **5 min** | **10 min** | **15 min** | **30 min** | **45 min** | **60 min** |
| Prep. Example 14 | 0 | 90.11 | 92.37 | 92.34 | 92.60 | 94.02 | 93.37 |
| Prep. Example 15 | 0 | 88.77 | 96.06 | 89.16 | 92.28 | 92.31 | 96.84 |
| Prep. Example 16 | 0 | 96.83 | 95.11 | 98.30 | 98.44 | 99.79 | 99.25 |
| Prep. Example 17 | 0 | 98.47 | 97.68 | 97.87 | 97.49 | 97.99 | 99.40 |
| Prep. Example 18 | 0 | 97.19 | 96.05 | 94.79 | 95.40 | 95.07 | 97.68 |
| Prep. Example 19 | 0 | 95.28 | 96.91 | 97.23 | 97.89 | 98.31 | 98.76 |

**[TABLE 16]**

| **Rosuvastatin (pH 6.8)** | | | | | | | |
|---|---|---|---|---|---|---|---|
| **Dissolution time** | **0 min** | **5 min** | **10 min** | **15 min** | **30 min** | **45 min** | **60 min** |
| Prep. Example 14 | 0 | 89.34 | 93.12 | 94.68 | 95.86 | 96.73 | 97.45 |
| Prep. Example 15 | 0 | 90.96 | 93.50 | 94.28 | 94.90 | 95.05 | 95.13 |
| Prep. Example 16 | 0 | 89.65 | 92.22 | 93.06 | 94.12 | 94.49 | 94.98 |
| Prep. Example 17 | 0 | 92.95 | 93.78 | 95.24 | 96.88 | 97.91 | 98.71 |
| Prep. Example 18 | 0 | 89.76 | 94.75 | 95.44 | 96.43 | 97.96 | 97.71 |
| Prep. Example 19 | 0 | 88.48 | 92.77 | 94.14 | 95.98 | 97.29 | 97.94 |

The formulations of Preparative Examples 14 to 19 are products obtained by adjusting raw drug substances and contents thereof in the formulation of Preparative Example 1. It could be determined that the dissolution rates of olmesartan, amlodipine and rosuvastatin components are substantially equal in specific eluates corresponding to separate ingredients despite changes in raw drug substances and contents thereof.

## Claims

1. A pharmaceutical composition in a single dosage form, comprising:
a composition comprising olmesartan medoxomil; a composition comprising amlodipine or a salt thereof; and a composition comprising rosuvastatin or a salt thereof,
wherein the above compositions are mixed together and formulated into the single dosage form of pharmaceutical composition, and
wherein one or more of the compositions is comprised in the form of granulates or pellets.

2. The pharmaceutical composition according to claim 1, wherein the pharmaceutical composition has a dosage form selected from: a single layer tablet; a capsulecomprising one or more selected from the group consisting of granulates, pellets and powder; or a granulate formulation.

3. The pharmaceutical composition according to claim 1, wherein the composition comprising olmesartan medoxomil in the pharmaceutical composition is comprised in the form of granulates.

4. The pharmaceutical composition according to claim 1, wherein the pharmaceutical composition has a dosage form selected from:: a single layer tablet in which granulates comprising olmesartan medoxomil and amlodipine or a salt thereof and granulates comprising rosuvastatin or a salt thereof are mixed together; a single layer tablet in which granulates comprising olmesartan medoxomil and amlodipine or a salt thereof and a composition comprising rosuvastatin or a salt thereof are mixed together; a single layer tablet in which olmesartan medoxomil granulates, a composition comprising amlodipine or a salt thereof and a composition comprising rosuvastatin or a salt thereof are mixed together; a single layer tablet in which olmesartan medoxomil granulates, a composition comprising amlodipine or a salt thereof and granulates comprising rosuvastatin or a salt thereof are mixed together; or a single layer tablet in which olmesartan medoxomil granulates and granulates comprising amlodipine or a salt thereof and rosuvastatin or a salt thereof are mixed together.

5. The pharmaceutical composition according to claim 1, wherein the pharmaceutical composition has a dosage form selected from: a capsule in which pellets comprising olmesartan medoxomil and amlodipine or a salt thereof, and pellets or powder comprising rosuvastatin or a salt thereof are comprised; a capsule in which granulates comprising olmesartan medoxomil and amlodipine or a salt thereof, and pellets or powder comprising rosuvastatin or a salt thereof are comprised; a capsule in which pellets comprising olmesartan medoxomil and amlodipine or a salt thereof, and granulates comprising rosuvastatin or a salt thereof are comprised; a capsule in which granulates comprising olmesartan medoxomil and amlodipine or a salt thereof, and granulates comprising rosuvastatin or a salt thereof are comprised; or a capsule in which olmesartan medoxomil granulates, and granulates comprising amlodipine or a salt thereof and rosuvastatin or a salt thereof are comprised.

6. The pharmaceutical composition according to claim 1, wherein the pharmaceutical composition has a dosage form selected from: a granulate formulation in which granulates comprising omesartan medoxomil and amlodipine or a salt thereof and granulates comprising rosuvastatin or a salt thereof are comprised; or a granulate formulation in which olmesartan medoxomil granulates and granulates comprising amlodipine or a salt thereof and rosuvastatin or a salt thereof arecomprised.

7. The pharmaceutical composition according to claim 1, wherein the pharmaceutical composition comprises two or more disintegrants selected from the group consisting of pre-gelatinized starch, sodium croscarmellose, crospovidone, calcium carboxymethyl cellulose, sodium starch glycolate, copovidone and combined silicate.

8. The pharmaceutical composition according to claim 7, wherein the disintegrant is comprised in an amount of 10 to 60 parts by weight ("wt. parts") to total 100 wt. parts of the pharmaceutical composition.

9. The pharmaceutical composition according to claim 1, wherein the pharmaceutical composition comprises, with regard to total 100 wt. parts of the pharmaceutical composition, 4 to 40 wt. parts of one or more disintegrants selected from pre-gelatinized starch and sodium starch glycolate, as well as 2 to 30 wt. parts of one or more disintegrants selected from the group consisting of sodium croscarmellose, crospovidone, copovidone, low-substituted hydroxylpropyl cellulose and calcium carboxymethyl cellulose.

10. The pharmaceutical composition according to claim 7, wherein the pharmaceutical composition comprises calcium hydrophosphate hydrate, anhydrous calcium phosphate, calcium carbonate or a mixture thereof.

11. The pharmaceutical composition according to claim 10, wherein calcium hydrophosphate hydrate, anhydrous calcium phosphate, calcium carbonate or a mixture thereof is comprised in an amount of 3 to 20 wt. parts to total 100 wt. parts of the pharmaceutical composition.

12. The pharmaceutical composition according to claim 1, wherein the pharmaceutical composition is a single layer tablet coated with a coating agent.

13. The pharmaceutical composition according to claim 12, wherein the pharmaceutical composition is a single layer tablet coated with polyvinylalcohol, polyvinylalcohol copolymer or hydroxypropyl methylcellulose (HPMC).

14. The pharmaceutical composition according to claim 1, wherein the salt of amlodipine is amlodipine besylate.

15. The pharmaceutical composition according to claim 1, wherein the salt of rosuvastatin is rosuvastatin calcium.

16. The pharmaceutical composition according to claim 1, wherein olmesartan medoxomil has D(90) of 5 to 45 µm.

17. The pharmaceutical composition according to claim 1, wherein amlodipine or a salt thereof has D(90) of 5 to 100 µm.

18. The pharmaceutical composition according to claim 1, wherein rosuvastatin or a salt thereof has D(90) of 5 to 50 µm.

19. The pharmaceutical composition according to claim 1, wherein the pharmaceutical composition is used for treating or preventing hypertension or hyperlipidemia.

20. The pharmaceutical composition according to claim 1, wherein an dissolution rate of amlodipine or a salt thereof is equivalent to that of amlodipine or a salt thereof in Sebica^{™} tablet.

21. The pharmaceutical composition according to claim 1, wherein an dissolution rate of rosuvastatine or a salt thereof is equivalent to that of rosuvastatin or a salt thereof in Cresto^{™} tablet.

22. The pharmaceutical composition according to claim 1, wherein olmesartan medoxomil and amlodipine or a salt thereof in the pharmaceutical composition exhibit an area under curve (AUC) of blood concentration-time and the maximum serum concentration (Cₘₐₓ) at bioequivalent levels as compared to Sebica^{™} tablet having the same active ingredient dose.

23. The pharmaceutical composition according to claim 1, wherein the rosuvastatin calcium salt in the pharmaceutical composition exhibits an area under curve (AUC) of blood concentration-time and the maximum serum concentration (Cₘₐₓ) at bioequivalent levels as compared to Cresto^{™} tablet having the same active ingredient dose.

24. The pharmaceutical composition according to claim 1, wherein the pharmaceutical composition is used for administration to a patient who requires a combination therapy of amlodipine and olmesartan medoxomil and, at the same time, should be administered with rosuvastatin.

25. The pharmaceutical composition according to claim 1, wherein the pharmaceutical composition comprises olmesartan medoxomil, amlodipine and rosuvastatin in the following doses, on the basis of a unit dosage form:
40 mg of olmesartan medoxomil, 10 mg of amlodipine or a salt thereof, and 20 mg of rosuvastatin or a salt thereof;
40 mg of olmesartan medoxomil, 10 mg of amlodipine or a salt thereof, and 10 mg of rosuvastatin or a salt thereof;
40 mg of olmesartan medoxomil, 5 mg of amlodipine or a salt thereof, and 10 mg of rosuvastatin or a salt thereof;
40 mg of olmesartan medoxomil, 5 mg of amlodipine or a salt thereof, and 5 mg of rosuvastatin or a salt thereof;
20 mg of olmesartan medoxomil, 5 mg of amlodipine or a salt thereof, and 10 mg of rosuvastatin or a salt thereof;
20 mg of olmesartan medoxomil, 5 mg of amlodipine or a salt thereof, and 5 mg of rosuvastatin or a salt thereof;
20 mg of olmesartan medoxomil, 5 mg of amlodipine or a salt thereof, and 2.5 mg of rosuvastatin or a salt thereof;
40 mg of olmesartan medoxomil, 2.5 mg of amlodipine or a salt thereof, and 20 mg of rosuvastatin or a salt thereof;
40 mg of olmesartan medoxomil, 2.5 mg of amlodipine or a salt thereof, and 10 mg of rosuvastatin or a salt thereof;
20 mg of olmesartan medoxomil, 2.5 mg of amlodipine or a salt thereof, and 10 mg of rosuvastatin or a salt thereof;
20 mg of olmesartan medoxomil, 2.5 mg of amlodipine or a salt thereof, and 5 mg of rosuvastatin or a salt thereof; or
20 mg of olmesartan medoxomil, 2.5 mg of amlodipine or a salt thereof, and 2.5 mg of rosuvastatin or a salt thereof.
